# EUROPÄISCHE PATENTSCHRIFT

(11) **EP 2 539 841 B1**
(45) Veröffentlichungstag und Bekanntmachung des Hinweises auf die Patenterteilung: **04.04.2018**
(21) Anmeldenummer: 11705556.6
(22) Anmeldetag: 24.02.2011
(51) Int. Cl.: G06F 19/00

(54) **SYSTEM UND VERFAHREN ZUR STEUERUNG EINER DATENÜBERTRAGUNG AN UND/ODER VON EINER MEHRZAHL VON MEDIZINISCHEN GERÄTEN**
SYSTEM AND METHOD FOR CONTROLLING A DATA TRANSMISSION TO AND/OR FROM A PLURALITY OF MEDICAL DEVICES
SYSTÈME ET PROCÉDÉ DE COMMANDE D'UNE TRANSMISSION DE DONNÉES VERS ET/OU DEPUIS UNE PLURALITÉ D'APPAREILS MÉDICAUX

(30) Priorität: 26.02.2010 DE 102010009540
(43) Veröffentlichungstag der Anmeldung: 02.01.2013
(73) Patentinhaber: B. Braun Melsungen AG, 34212 Melsungen (DE)
(72) Erfinder: SCHMOLL, Horst, 34302 Guxhagen (DE); PÄTZOLD, Matthias, 34212 Melsungen (DE)
(74) Vertreter: Winter, Brandl, Fürniss, Hübner, Röss, Kaiser, Polte - Partnerschaft mbB
(86) Internationale Anmeldenummer: PCT/EP2011/052728
(87) Internationale Veröffentlichungsnummer: WO 2011/104296

(56) Entgegenhaltungen:
- EP-A2- 1 515 496
- WO-A1-2006/067271
- US-A1- 2006 031 378
- US-A1- 2009 238 087
- V R Mamkin ET AL: "CAN BUS GATEWAY FOR DATA ACQUISITION AND CONTROL", , 10 September 2006 (2006-09-10), XP055340665, Retrieved from the Internet: URL:https://accelconf.web.cern.ch/accelcon f/r06/PAPERS/MODP01.PDF [retrieved on 2017-01-31]

## Beschreibung

Die Erfindung betrifft ein System und ein Verfahren zur Steuerung einer Datenübertragung an und/oder von eine/einer Mehrzahl von medizinischen Geräten.

In Kliniken bzw. Krankenhäusern werden häufig Netzwerke zur Übertragung von Daten zur Verfügung gestellt, die dafür vorgesehen sind, eine Datenkommunikation mit einer Mehrzahl an medizinischen Geräten auf der einen Seite und einem Server bzw. einer Servereinrichtung auf der anderen Seite zur Verfügung zu stellen. Derartige Netzwerke haben zur Aufgabe, dass Datenübertragungen zeitnah stattfinden. Dies betrifft sowohl Daten, die an den medizinischen Geräten, welche häufig mit Patienten, die ein Patientenbett belegen, verbunden sind, erzeugt werden und an die zentrale Servereinrichtung gesendet werden müssen, um die Daten zu überwachen, auszuwerten und anzuzeigen, als auch Daten, die von der Servereinrichtung aus zentral an die verschiedenen medizinischen Geräte gesendet werden sollen. Diese Datenübertragung muss zeitnah (near real time) und störungsfrei erfolgen, welches häufig schon allein dadurch nicht sichergestellt ist, da sämtliche medizinische Geräte auf ein gemeinsames Netzwerk zugreifen und hierdurch häufig eine Überlastung des Netzwerkes stattfindet.

Zudem kann durch eine derartige Überlastung eine Unterbrechung in der Datenübertragung erfolgen und somit keine störungsfreie Datenübermittlung durchgeführt werden. Dies führt häufig dazu, dass in den medizinischen Geräten große Zwischenspeicher angeordnet werden, um bei Ausfall oder Überlastung des Netzwerkes die Daten, die momentan in dem medizinischen Gerät erzeugt werden oder zuvor empfangen worden sind, zwischenzuspeichern.

Ein Auslesen dieses Zwischenspeichers wird erst dann wieder ermöglicht, wenn eine feste Datenübertragungsverbindung mit der Servereinrichtung, beispielsweise nach Abbau einer Netzwerküberlastung, aufgebaut worden ist.

Aus dem Stand der Technik sind die Druckschriften US 2009/238087 A1, US 2006/031378 A1, WO 2006/067271 A1 und EP 1 515 496 A2 bekannt.

Demzufolge liegt der vorliegenden Erfindung die Aufgabe zugrunde, ein System und ein Verfahren zur Steuerung einer Datenübertragung an und/oder von eine/einer Mehrzahl von medizinischen Geräten zur Verfügung zu stellen, welche eine störungsfreie, fortlaufende Übertragung von Daten zwischen den medizinischen Geräten einerseits und einer Servereinrichtung andererseits sicherstellen, ohne dass ein Datenverlust bei zeitnaher Übertragung der Daten und eine Überlastung eines Netzwerkes stattfindet.

Diese Aufgabe wird systemseitig durch die Merkmale des Patentanspruches 1 und verfahrensseitig durch die Merkmale des Patentanspruches 6 gelöst.

Ein wesentlicher Punkt der Erfindung liegt darin, dass bei einem System zur Steuerung einer Datenübertragung an und/oder von eine/einer Mehrzahl von medizinischen Geräten, wobei die Mehrzahl von medizinischen Geräten in einzelne Gruppen mit jeweils mindestens einem medizinischen Gerät unterteilt ist, jede Gruppe an medizinischen Geräten auf einer ersten Datenübertragungshierarchieebene mit einer auf einer zweiten Datenübertragungshierarchieebene angeordneten Kommunikationseinrichtung für die Übertragung, Speicherung und Steuerung von Daten über jeweils ein erstes Netzwerk direkt verbunden ist und Mittel vorgesehen sind, die dazu ausgebildet sind, dass eine Mehrzahl dieser Kommunikationseinrichtungen auf einer dritten Datenübertragung mit einer auf einer dritten Datenübertragungshierarchieebene angeordneten gemeinsamen zentralen Servereinrichtung zum Speichern, zur Steuerung und zur Datenübertragung Daten austauscht. Derartige Mittel können ein zweites Netwerk sein, welches die Kommunikationseinrichtung direkt mit der Servereinrichtung verbindet. Alternativ oder zusätzlich können derartige Mittel externe Speichermedien, wie beispielsweise USB-Sticks oder Speicherkarten, darstellen, die zur Übertragung von Daten dienen können.

Durch ein derartiges System zur Steuerung einer Datenübertragung wird vorteilhaft ermöglicht, dass aufgrund der mehrstufigen Anordnung der einzelnen an der Datenübertragung beteiligten Systemkomponenten nicht nur die Netzwerkbelastung mit Daten minimiert und damit eine Überlastung der beteiligten Netzwerke verhindert wird, sondern auch eine störungsfreie Datenübertragung zwischen den medizinischen Geräten einerseits und der Servereinrichtung andererseits sichergestellt wird. Denn durch die Verbindung der einzelnen medizinischen Geräte, die in die Gruppen aufgeteilt sind, mit jeweils einer Kommunikationseinrichtung, die im Übrigen derart ausgebildet ist, dass sie autark, also unabhängig von der Servereinrichtung, mit den medizinischen Geräten, welches vorzugsweise Infusionspumpen sind, kommunizieren kann und auch Daten speichern kann, wird eine direkte Verbindung zwischen jeweils einer Kommunikationseinrichtung einerseits und einem medizinischen Gerät andererseits als exklusive Verbindung des ersten Netzwerkes geschaffen.

Dies hat zur Folge, dass beispielsweise bei der Verwendung eines Netzwerkes vom Typ CAN, keine zeitlichen und mengenmäßen Überschneidungen von Daten bzw. Datenpaketen zwischen medizinischen Geräten und einer zentral angeordneten Servereinrichtung stattfinden kann, da die exklusive Direktleitung zwischen der Kommunikationseinrichtung und einem der medizinischen Geräte die Latenzzeiten und Datenverluste bei der Übertragung von Daten reduziert und nahezu ausschließt. Es werden hierdurch nicht nur Wiederholschleifen, wie sie häufig für eine erfolgreiche Übertragung von Daten über ein Netzwerk, welches eine Vielzahl von medizinischen Geräten gleichzeitig versorgen soll, erfolgen muss, sondern auch Update - Abbrüche beim Updaten der Software der medizinischen Geräte verhindert.

Zudem kann aufgrund dieser Direktleitung zwischen der Kommunikationseinrichtung und der medizinischen Geräte eine störungsfreie Übertragung der Daten von dem medizinischen Gerät zur Kommunikationseinrichtung oder vice versa erfolgen. Dies kann sowohl geräte-spezifische und patientenbezogene Daten von Patienten, die mit den medizinischen Geräten, wie Insulinpumpen, in Verbindung stehen, als auch medikamentenspezifische Daten, die von der Servereinrichtung an die medizinischen Geräte versandt werden soll, betreffen.

Hierfür weist die zentrale Servereinrichtung eine Anwendungsservereinheit und eine Speichereinheit auf, wobei die Anwendungsservereinheit mit Anwendungsbedieneinheiten auf einer vierten Datenübertragungshierarchieebene mittels eines dritten Netzwerkes verbunden ist. Somit ist eine Mehrzahl an Anwendungsbedieneinheiten auf einer weiteren Datenübertragungshierarchieebene dieser kaskadisch oder mehrstufig aufgebauten Kommunikationsstruktur angeordnet, woraus sich eine Vielzahl an lose miteinander kommunizierender Einrichtungen, Einheiten und medizinischer Geräte auf verschiedenen Stufen dieser Kommunikationsstruktur ergeben, die relativ eigenständig funktionieren und agieren können, um die Kommunikation mit den benachbarten Einrichtungen/Einheiten/medizinischen Geräten der nächstniedrigeren oder nächsthöheren Stufe weiterhin aufrecht zu erhalten. Dies ermöglicht eine Reduzierung der Anfälligkeit gegenüber Kommunikationsstörungen, wie sie beispielsweise häufig bei der Verwendung es Wireless LAN (WLAN) mit einem Server, der über ein derartiges Netzwerk direkt mit den medizinischen Geräten in der gesamten Klinik verbunden ist, auftreten können.

Jede Kommunikationseinrichtung ist hierfür mit mindestens einer ersten Speichereinheit und einer ersten Steuereinheit ausgestattet, um von den medizinischen Geräten der Gruppe kommende und empfangende Daten, insbesondere gerätespezifische und patientenbezogene Daten von Patienten, die mit den medizinischen Geräten in Verbindung stehen, zwischenzuspeichern und gebündelt an die Speicherservereinheit weiterzugeben.

Ebenso ist die erste Speichereinheit und die erste Steuereinheit einer jeder Kommunikationseinrichtung dafür geeignet, von der Speicherservereinheit oder Anwendungsservereinheit kommende und empfangende Daten, insbesondere medikamentenspezifische Daten, zwischenzuspeichern und auf Abfrage oder bei Bedarf an ausgewählte medizinische Geräte weiterzusenden. Dies ermöglicht, dass Daten sowohl von der Kommunikationseinrichtung zu den medizinischen Geräten als auch von den medizinischen Geräten zu der Kommunikationseinrichtung gesendet werden können, wobei sämtliche Daten innerhalb einer jeden Kommunikationseinrichtung zwischengespeichert werden können und gegebenenfalls auch direkt aus dieser Kommunikationseinrichtung ohne Zuhilfenahme einer Servereinrichtung ausgelesen oder in diese geschrieben werden können. Hierdurch ist ein nahezu unabhängiges erstes Netzwerk zwischen einer Gruppe von medizinischen Geräten einerseits und mindestens einer Kommunikationseinrichtung andererseits möglich.

Ebenso ist das zweite Netzwerk, welches zwischen einer Mehrzahl derartiger Kommunikati-onseinrichtungen einerseits und der Servereinrichtung aufgebaut ist, als von den anderen Netzwerken unabhängiges Netzwerkwerk zu betrachten. Insofern kann eine Datenübertragung von der Servereinrichtung zu den einzelnen Kommunikationseinrichtungen, die beispielsweise notwendig ist, um Medikamentendaten, die über die Servereinrichtung in neuer Form zur Verfügung stehen, an die einzelnen Kommunikationseinrichtungen und damit die medizinischen Geräte weiterzugeben, zunächst mittels des zweiten Netzwerkes erfolgen und innerhalb der einzelnen Kommunikationseinrichtungen können diese Daten zwischengespeichert werden, um anschließend bei Bedarf und bei geeignetem Betriebszustand der medizinischen Geräte an diese weitergeleitet zu werden. Dies kann beispielsweise dann vorliegen, wenn das als Insulinpumpe ausgebildete medizinische Gerät gerade keine Medikamentenverabreichung in Form von Insulin an den Patienten.

Auch das dritte Netzwerk zwischen der Servereinrichtung und den Anwendungsbedieneinheiten, die entfernt von der Servereinrichtung angeordnet sein können, ist als autark zu betrachten. Dieses unabhängige dritte Netzwerk kann beispielsweise mittels eines Web-Interfaces und eines Web-Browsers die Anwendungsbedieneinheiten mit der Anwendungsservereinheit verbinden und somit eine Bedienung, Steuerung, ein Uploaden und ein Auslesen der auf der Servereinrichtung gespeicherten Daten ermöglichen.

Die auf der Servereinrichtung gespeicherten Daten, die vorrangig in der Speicherservereinheit abgespeichert sind, können ebenso visuell dargestellt werden, wobei diese Darstellung sowohl auf der Servereinrichtung als auch in einer oder mehreren Anwendungsbedieneinheiten, die entfernt von der Servereinrichtung angeordnet sind, erfolgen kann.

Ein erfindungsgemäßes Verfahren zur Steuerung der Datenübertragung an und/oder von der Mehrzahl von medizinischen Geräten zeichnet sich dadurch aus, dass jede Gruppe an medizinischen Geräten auf einer ersten Datenübertragungshierarchieebene von/an eine auf einer zweiten Datenübertragungshierarchieebene angeordnete Kommunikationseinrichtung Daten über jeweils ein erstes Netzwerk überträgt und am Empfängerort oder am Senderort speichert und gegebenenfalls zur Steuerung des Gerätes bzw. der Einrichtung verwendet. Diese kann gerätespezifische Konfigurationsdaten, gerätespezifische Medikamentendaten, gerätespezifische Softwareprogramme sowie das Sammeln und Auswerten von gerätespezifischen Statusinformationen und das Sammeln und Auswerten von gerätespezifischen Therapieinformationen betreffen.

Die auf der ersten Datenübertragungshierarchieebene agierenden Geräte, die im Übrigen ebenso nahezu netzunabhängig arbeiten und damit autark funktionieren, werden somit von den Kommunikationseinrichtungen über Konfigurationsdateien und Kommandos gesteuert und geben anders herum Betriebs-, Status- und medizinische Daten an die Kommunikationseinrichtung weiter.

Die Kommunikationseinrichtungen auf der zweiten Datenübertragungshierarchieebene sind ebenso nahezu netzwerkunabhängig und insbesondere von der Servereinrichtung autark arbeitend ausgebildet und empfangen Konfigurationsdateien und Kommandos von der Servereinrichtung bei Gelegenheit, um diese selbstständig an die an die jeweilige Kommunikationseinrichtung angeschlossenen medizinischen Geräte weiterzugeben.

Ebenso werden anders herum Betriebs-, Status- und medizinische Daten sämtlicher angeschlossenen medizinischer Geräte empfangen und zwischengespeichert, um sie dann gebündelt bzw. konzentriert an die Servereinrichtung auf der dritten Datenübertragungshierarchieebene weiterzugeben.

Zudem wird das zeitliche und funktionelle Verhalten der Kommunikationseinrichtungen vorzugsweise über die zentrale Servereinrichtung auf der dritten Datenübertragungshierarchieebene durch Übertragung derartiger Steuerungsdaten gesteuert.

Die auf der dritten Datenübertragungshierarchieebene angeordnete Servereinrichtung ist vorzugsweise innerhalb der Klinik zentral angeordnet und wird von den Anwendungsbedieneinheiten auf der vierten Datenübertragungshierarchieebene gesteuert. Eine derartige Steuerung kann veranlassen, dass gerätespezifische Konfigurationsdaten und Kommandos an ausgewählte Kommunikationseinrichtungen der zweiten Datenübertragungshierarchieebene weitergeleitet werden. Ebenso werden Betriebs-, Status- und medizinische Daten sämtlicher angeschlossener Kommunikationseinrichtungen der zweiten Datenübertragungshierarchieebene innerhalb dieser dritten Übertragungsstufe, also innerhalb der Servereinrichtung, zwischengespeichert.

Die in der vierten Datenübertragungshierarchieebene angeordneten Anwendungsbedieneinheiten erzeugen die Konfigurationsdateien und Kommandos automatisiert oder mit Hilfe von klinischen Personal. Zudem führen sie eine Auswahl der anzusprechenden Kommunikationseinheiten durch und definieren das funktionale und zeitliche Verhalten der Kommunikationseinrichtungen, indem sie die hierfür notwendigen Daten bzw. Informationen in der Servereinrichtung gemäß der dritten Datenübertragungshierarchieebene zur Verfügung zu stellen.

Die Anwendungsbedieneinheiten können auch die Betriebs-, Status- und medizinischen Daten, die in der Servereinrichtung zwischengespeichert sind, in geeigneter Weise auswerten, konzentrieren und darstellen.

Die Anwendungsservereinheit wird von den Anwendungsbedieneinheiten bedient, gesteuert sowie datenschreib- und datenlesegeregelt.

Auch bei dem erfindungsgemäßen Verfahren werden in jeder Kommunikationseinrichtung in mindestens einer ersten Speichereinheit und mittels einer ersten Steuereinheit empfangene Daten, die von den medizinischen Geräten der Gruppe gesendet werden, insbesondere gerätespezifische und patientenbezogene Daten von Patienten, die mit den medizinischen Geräten in Verbindung stehen, zwischengespeichert und gebündelt und an die Speicherservereinheit weitergesendet.

Ebenso werden mittels der ersten Speichereinheit und der ersten Steuereinheit die Daten, insbesondere medikamentenspezifische Daten, von der Speicherservereinheit oder der Anwendungsservereinheit empfangen und zwischengespeichert, um sie auf Abfrage oder bei Bedarf an ausgewählte medizinische Geräte weiterzusenden. Dies kann beispielsweise dann vorliegen, wenn das medizinische Gerät sich in einem für das Updaten mit Daten geeigneten Betriebszustand befindet. Dann nimmt die Kommunikationseinrichtung das Übertragen und das Updaten der Daten in dem medizinischen Gerät selbstständig vor. Hierfür werden die Daten von sämtlichen medizinischen Geräten fortlaufend von der Kommunikationseinrichtung ausgelesen und innerhalb der Kommunikationseinrichtung zwischengespeichert.

Sobald diese zwischengespeicherten Daten, zu denen auch Betriebszustandsdaten der medizinischen Geräte gehören, anzeigen, dass ein geeigneter Betriebsmodus des medizinischen Gerätes vorliegt, findet ein Updaten einer Datenbank des medizinischen Gerätes durch die Kommunikationseinrichtung statt. Dies darf nicht in einem Betriebszustand erfolgen, während dem eine laufende Therapie durch das medizinische Gerät stattfindet.

Vorzugsweise wird das Vorliegen neuer Daten, wie auch neuer Konfigurationsdaten, innerhalb des medizinischen Gerätes visuell angezeigt.

Das erste Netzwerk ist vorzugsweise vom Typ CAN und das zweite sowie dritte Netzwerk sind vom Typ WLAN und/oder LAN.

Weitere vorteilhafte Ausführungsformen ergeben sich aus den Unteransprüchen.

Vorteile und Zweckmäßigkeiten sind der nachfolgenden Beschreibung mit der Zeichnung zu entnehmen. Hierbei zeigen:
Fig. 1 in einer schematischen Darstellung das mehrstufig aufgebaute erfindungsgemäße System; und
Fig. 2a und 2b in einem Flussdiagramm in Form einer Ausführungsform eine mögliche Anwendung des mehrstufigen erfindungsgemäßen Systems und Verfahren.

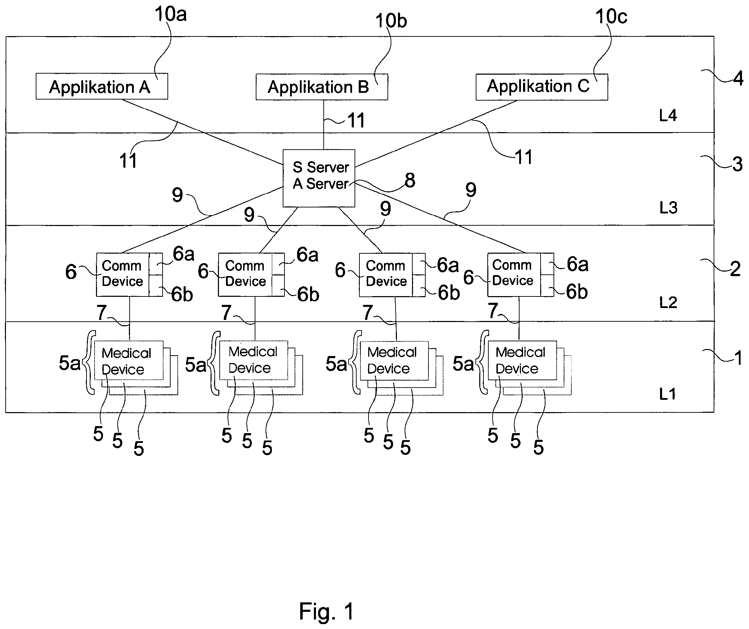

In Fig. 1 ist in einer schematischen Darstellung der Grundaufbau des erfindungsgemäßen Systems zur Durchführung des erfindungsgemäßen Verfahrens zur Steuerung einer Datenübertragung von einer Mehrzahl an medizinischen Geräten dargestellt.

Dieses System ermöglicht die Steuerung und Konfiguration einer Vielzahl von medizinischen Geräten ohne Datenübertragungsstörung und ohne Überlastung der damit verbundenen Netzwerke sowie eine Sammlung und Visualisierung von weiteren Daten, die von den medizinischen Geräten gesendet werden und zu Auswertungs- und Überwachungszwecken zentral gesammelt werden.

Es bestehen insgesamt vier Datenübertragungshierarchieebenen 1 - 4, wobei in der ersten Datenübertragungshierarchieebene 1 eine Vielzahl von medizinischen Geräten 5, wie Insulinpumpen, unterteilt in verschiedene Gruppen 5a angeordnet sind.

Jeweils eine Gruppe 5a an medizinischen Geräten ist einer Kommunikationseinrichtung 6 mit einer ersten Speichereinheit 6a und einer ersten Steuereinheit 6b zugeordnet. Dies ist mittels eines exklusiven Netzwerkes auf Basis von CAN gemäß dem Bezugszeichen 7 realisiert. Somit besteht zwischen der Kommunikationseinrichtung und jedem ihr zugeordneten medizinischen Gerät eine exklusive CAN-Verbindung 7.

Die Vielzahl an Kommunikationseinrichtungen 6 sind wiederum über ein zweites Netzwerk 9 mit einer zentral angeordneten Servereinrichtung 8 verbunden, wobei die Servereinrichtung 8 in eine Speicherservereinheit SS und eine Anwendungsservereinheit AS unterteilt sein kann.

Die Servereinrichtung 8 kann wiederum mittels eines dritten Netzwerkes 11 von der dritten Datenübertragungshierarchieebene 3 aus mit den auf der vierten Datenübertragungshierarchieebene 4 angeordneten Anwendungsbedieneinheiten 10a, 10b, 10c verbunden sein, welche unterschiedliche Anwendungen A, B, C ermöglichen.

Eine derartige mehrstufige Ausbildung des erfindungsgemäßen Systems zu Förderung der Datenübertragung ermöglicht eine störungsfreie und überlast-unanfällige Datenübertragung über die zur Verfügung stehenden Netzwerke.

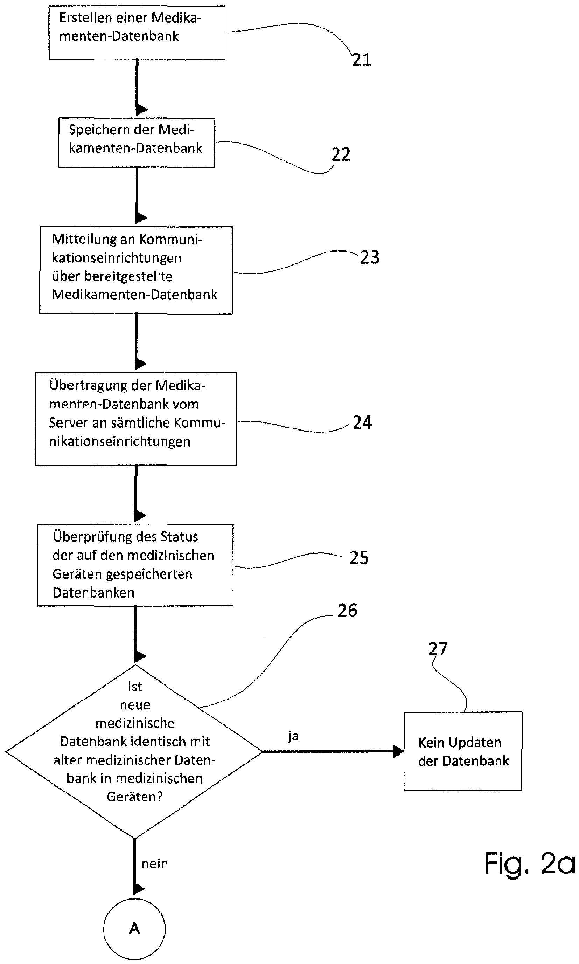

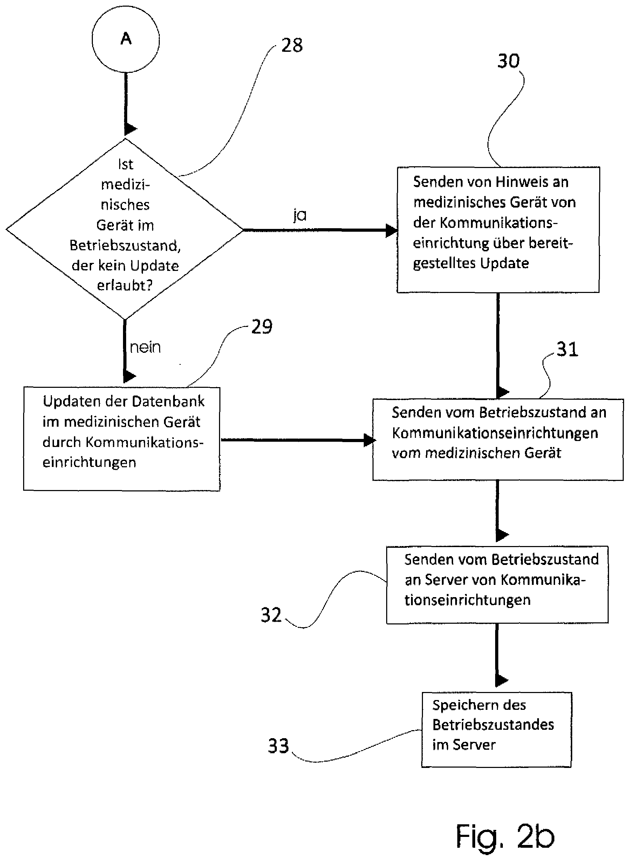

In Fig. 2a und 2b ist in einem Flussdiagramm eine Ausführungsform mit einer möglichen Realisierung des erfindungsgemäßen Verfahrens, zu welchem das erfindungsgemäße System notwendig ist, dargestellt.

Zunächst wird mittels einer der Anwendungsbedieneinheiten eine neue aktualisierte Medikamenten-Datenbank erstellt. Dies geschieht gemäß Schritt 21.

Anschließend findet durch eine Übersendung eines entsprechenden Steuersignals von der Anwendungsbedieneinheit an die Servereinrichtung 8 ein Speichern der Medikamenten-Datenbank gemäß Schritt 22 statt.

Gemäß Schritt 23 erhalten sämtliche Kommunikationseinrichtungen 6 die Mitteilung, dass eine neue Medikamenten-Datenbank in der Servereinrichtung bereitgestellt ist. Anschließend findet durch Abruf oder durch Übersendung der Daten für die neue Medikamenten-Datenbank bei Bedarf eine Übertragung der Medikamenten-Datenbanken vom Server an sämtliche Kommunikationseinrichtungen gemäß Schritt 24 statt. Dies erfolgt über das zweite Netzwerk, nachdem das dritte Netzwerk bei der Übertragung von den Anwendungsbedieneinheiten zu der Speichereinrichtung genutzt worden ist.

Die Kommunikationseinrichtungen erhalten laufend Daten von den an sie angeschlossenen medizinischen Geräten über den aktuellen Zustand bzw. Status der jeweiligen Medikamenten-Datenbank, welche momentan auf den jeweiligen medizinischen Geräten gespeichert sind.

Sobald diese Überprüfung gemäß Schritt 25 stattgefunden hat, wird in einem Schritt 26 durch Übersendung eines Abfragesignals von der Kommunikationseinrichtung an das jeweilige medizinische Gerät oder durch Überprüfung der aktuellen Daten, die innerhalb der Kommunikationseinrichtung zu dem jeweiligen Gerät vorliegen, abgefragt, ob die Daten zu der neuen Medikamenten- Datenbank identisch mit den Daten zu der bisherigen vorhandenen Medikamenten- Datenbank in dem jeweiligen medizinischen Gerät sind. Sofern dies der Fall ist, findet kein Updaten der Medikamenten- Datenbank gemäß Schritt 27 statt.

Sofern die Daten der Medikamenten-Datenbank nicht übereinstimmen, wird in einem weiteren Schritt 28 abgefragt, ob das betroffene medizinische Gerät einen Betriebszustand eingenommen hat, der ein Updaten der Datenbank erlaubt oder ob es sich momentan in einem Therapieverfahren befindet. Sofern ein Betriebszustand des medizinischen Gerätes vorhanden ist, der ein Updaten erlaubt, wird gemäß Schritt 29 ein Updaten der Datenbank des medizinischen Gerätes durch Übertragung der Medikamenten-Datenbank-Daten von der Kommunikationseinrichtung des medizinischen Geräts durchgeführt. Anschließend findet eine Ansteuerung des Abschnittes 31 statt, gemäß welchem der aktuelle Betriebszustand von dem medizinischen Gerät an die Kommunikationseinrichtung zugesandt wird.

Sofern der Betriebszustand momentan kein Updaten erlaubt, wird gemäß Schritt 30 ein Hinweis über das bereitgestellte Update von der Kommunikationseinrichtung an das medizinische Gerät gesendet. Das medizinische Gerät sendet in Antwort darauf gemäß Schritt 31 den aktuellen Betriebszustand an die Kommunikationseinrichtung, welche wiederum gemäß Schritt 32 im aktuellen Betriebszustand an den Server sendet, der gemäß Schritt 33 den aktuellen Betriebszustand speichert, um eine zentrale Datenbank über die Betriebszustände sämtlicher Geräte zu aktualisieren.

Die Kommunikationseinrichtung überprüft fortwährend den Betriebszustand der medizinischen Geräte (Schritt 28) und nimmt selbstständig ein Update vor, sobald der Betriebszustand eines medizinischen Gerätes ein Update erlaubt.

Gemäß dem Pfeil 34 findet somit eine wiederholte Abfrage des Betriebszustandes durch die Kommunikationseinrichtung solange statt, bis festgestellt wird, dass der Betriebszustand des medizinischen Gerätes gemäß Schritt 28 es erlaubt, dass ein Updaten des medizinischen Gerätes möglich ist.

Bei der Übertragung von Betriebs- und Statusinformationen der medizinischen Geräte an die Servereinrichtungen werden demzufolge zwei Stufen der Datenübertragung durchlaufen, nämlich der Übergang von der ersten Datenübertragungshierarchieebene zu der zweiten Datenübertragungshierarchieebene und der Übergang von der zweiten Datenübertragungshierarchieebene zu der dritten Datenübertragungshierarchieebene.

Aufgrund des von einem medizinischen Gerät exklusiv genutzten ersten Netzwerkes mit der Kommunikationseinrichtung ist eine laufende Abfrage des Betriebszustandes und einer evtl. stattfindenden Änderung von Betriebs- und Statusdaten des einzelnen medizinisch Gerätes mit hoher Übertragungsqualität und geringer Störung möglich. Innerhalb der Kommunikationseinrichtung werden diese Betriebs- und Statusdaten zwischengespeichert und aufbereitet, um sie anschließend gebündelt an die Servereinrichtung mittels des zweiten Netzwerkes weiterzusenden.

Die Kommunikationseinrichtung protokolliert hierbei, welche Daten wann an die Servereinrichtung mittels des zweiten Netzwerkes gesendet werden und ist hierdurch in der Lage die Übertragung der Daten an die Servereinrichtung auf die neu eingetroffenen bzw. empfangenen Daten zu beschränken.

Sofern eine Störung des zweiten Netzwerkes auftreten sollte, sind sämtliche Daten für vorbestimmbare Zeitspannen innerhalb der Kommunikationseinrichtungen zwischengespeichert und werden bei erneuter Verfügbarkeit des zweiten Netzwerkes vollständig an die Servereinrichtung übertragen.

Gemäß einer Weiterbildung der Erfindung ist es möglich, dass Daten auch von der Kommunikationseinrichtung direkt an andere Drittsysteme gesendet werden, ohne dies über die Servereinrichtung geschehen zu lassen. Hierfür können auch Protokollanpassungen innerhalb der Kommunikationseinrichtung stattfinden.

Aufgrund der Reduzierung der zu übertragenden Daten von jeder Kommunikationseinrichtung 6 zu der Servereinrichtung auf diejenigen Daten, die bisher noch nicht übertragen worden sind, kann eine Überlastung des zweiten Netzwerkes 9 ausgeschlossen werden. Ebenso werden Datenverlust und Latenzzeiten innerhalb des ersten Netzwerkes 7 aufgrund der direkten Verbindung zwischen einer jeden Kommunikationseinrichtung 6 und jedem medizinischen Gerät 5 ausgeschlossen, da in jedem medizinischen Gerät 5 dessen gesamtes Protokoll samt Daten abgespeichert ist. Eine Abspeicherung von Daten innerhalb der Kommunikationseinrichtungen 6 für den Fall von Netzausfällen ist hierdurch nicht mehr notwendig.

### Bezugszeichenliste

1 , 2, 3, 4 Datenübertragungshierarchieebene
5, 5a medizinische Geräte
6 Kommunikationseinrichtung
6a Speichereinheit
6b Steuereinheit
7 erstes Netzwerk
8 zentrale Servereinrichtung
9 zweites Netzwerk
10a - 10c Anwendungsbedieneinheiten
11 drittes Netzwerk
21 -34 Verfahrensschritte
AS Anwendungsservereinheit
SS Speichereinheit

## Patentansprüche

1. System zur Steuerung einer Datenübertragung an und/oder von eine/einer Mehrzahl von medizinischen Geräten (5), wobei die Mehrzahl von medizinischen Geräten (5) in einzelne Gruppen (5a) mit jeweils mindestens einem medizinischen Gerät (5) unterteilt ist, wobei jede Gruppe (5a) an medizinischen Geräten (5) auf einer ersten Datenübertragungshierarchieebene (1) mit einer auf einer zweiten Datenübertragungshierarchieebene (2) angeordneten Kommunikationseinrichtung (6) für die Übertragung, Speicherung und Steuerung von Daten über jeweils ein erstes Netzwerk (7) direkt verbunden ist, wobei das erste Netzwerk (7) eine Direktleitung zwischen der Kommunikationseinrichtung (6) und den medizinischen Geräten (5) ist, und Mittel vorgesehen sind, die dazu ausgebildet sind, dass eine Mehrzahl dieser Kommunikationseinrichtungen (6) mit einer auf einer dritten Datenübertragungshierarchieebene (3) angeordneten gemeinsamen zentralen Servereinrichtung (8) zum Speichern, zur Steuerung und zur Datenübertragung Daten austauscht, wobei die Mittel ein zweites Netzwerk (9) sind, welches autark sowie unabhängig von dem ersten Netzwerk (7) ist, und welches die Kommunikationseinrichtungen (6) mit der auf der dritten Datenübertragungshierarchieebene (3) angeordneten gemeinsamen zentralen Servereinrichtung (8) direkt verbindet, wobei jede Kommunikationseinrichtung (6) mindestens eine erste Speichereinheit (6a) und eine erste Steuereinheit (6b) aufweist, um von den medizinischen Geräten (5) der Gruppe (5a) kommende und empfangene Daten, insbesondere gerätespezifische und patientenbezogene Daten von Patienten, die mit den medizinischen Geräten (5) in Verbindung stehen, zwischenzuspeichern und gebündelt an eine in der zentralen Servereinrichtung (8) vorgesehene Speicherservereinheit (SS) weiterzusenden, und wobei die Kommunikationseinrichtung (6) einen Betriebszustand eines jeden medizinischen Geräts (5), welches mit der Kommunikationseinrichtung (6) verbunden ist, laufend abfragt und fortwährend überprüft und ein Updaten einer Datenbank des jeweiligen medizinischen Geräts (5) selbstständig vornimmt, sobald der Betriebszustand des medizinischen Geräts (5) ein Updaten erlaubt.

2. System nach Anspruch 1, **dadurch gekennzeichnet, dass** die zentrale Servereinrichtung (8) neben der Speicherservereinheit (SS) eine Anwendungsservereinheit (AS) umfasst, wobei die Anwendungsservereinheit (AS) mit Anwendungsbedieneinheiten (10a, 10b, 10c) auf einer vierten Datenübertragungshierarchieebene (4) mittels eines dritten Netzwerkes (11) verbunden ist.

3. System nach Anspruch 2, **dadurch gekennzeichnet, dass** die erste Speichereinheit (6a) und die erste Steuereinheit (6b) der Kommunikationseinrichtung (6) dafür geeignet sind, von der Speicherservereinheit (SS) oder der Anwendungsservereinheit (AS) kommende und empfangene Daten, insbesondere medikamentenspezifische Daten, zwischenzuspeichern und auf Abfrage oder bei Bedarf an ausgewählte medizinische Geräte (5) weiterzusenden.

4. System nach einem der vorangegangenen Ansprüche 2 oder 3, **dadurch gekennzeichnet, dass** das dritte Netzwerk (11) mittels eines Web-Interfaces und eines Web-Browsers die Anwendungsbedieneinheiten (10a , 10b , 10c) mit der Anwendungsservereinheit (AS) verbindet.

5. System nach einem der vorangegangenen Ansprüche, **dadurch gekennzeichnet, dass** die medizinischen Geräte (5) Insulinpumpen sind.

6. Verfahren zur Steuerung einer Datenübertragung an und/oder eine/einer Mehrzahl von medizinischen Geräten (5), wobei die Mehrzahl an medizinischen Geräten (5) in einzelne Gruppen (5a) mit jeweils mindestens einem medizinischen Gerät (5) unterteilt werden, wobei jede Gruppe (5a) an medizinischen Geräten (5) auf einer ersten Datenübertragungshierarchieebene Daten von/an einer/eine auf einer zweiten Datenübertragungshierarchieebene (2) angeordneten Kommunikationseinrichtung (6) über jeweils ein erstes Netzwerk (7) überträgt und steuert, wobei das erste Netzwerk (7) eine Direktleitung zwischen der Kommunikationseinrichtung (6) und den medizinischen Geräten (5) ist, und eine Mehrzahl dieser Kommunikationseinrichtungen (6) Daten von/an einer/eine auf einer dritten Datenübertragungshierarchieebene (3) angeordneten gemeinsamen zentralen Servereinrichtung (8) Daten über ein zweites Netzwerk (9), welches autark sowie unabhängig von dem ersten Netzwerk (7) ist, überträgt und steuert, wobei in jeder Kommunikationseinrichtung (6) in mindestens einer ersten Speichereinheit (6a) und mittels einer ersten Steuereinheit (6b) empfangene Daten, die von den medizinischen Geräten (5) der Gruppe (5a) gesendet werden, insbesondere gerätespezifische und patientenbezogene Daten von Patienten, die mit den medizinischen Geräten (5) in Verbindung stehen, zwischengespeichert und gebündelt an eine in der zentralen Servereinrichtung (8) vorgesehene Speicherservereinheit (SS) weitergesendet werden, und wobei die Kommunikationseinrichtung (6) einen Betriebszustand eines jeden medizinischen Geräts (5), welches mit der Kommunikationseinrichtung (6) verbunden ist, laufend abfragt und fortwährend überprüft und ein Updaten einer Datenbank des jeweiligen medizinischen Geräts (5) selbstständig vornimmt, sobald der Betriebszustand des medizinischen Geräts (5) ein Updaten erlaubt.

7. Verfahren nach Anspruch 6, **dadurch gekennzeichnet, dass** die zentrale Servereinrichtung (8) neben der Speicherservereinheit (SS) mit einer Anwendungsservereinheit (AS) ausgestattet ist, wobei die Anwendungsservereinheit (AS) mit Anwendungsbedieneinheiten (10a, 10b, 10c) auf einer vierten Datenübertragungshierarchieebene (4) über ein drittes Netzwerk (11) verbunden ist und von den Anwendungsbedieneinheiten bedient, gesteuert sowie datenschreib- und datenlesegeregelt wird.

8. Verfahren nach Anspruch 7, **dadurch gekennzeichnet, dass** die erste Speichereinheit (6a) und die erste Steuereinheit (6b) der Kommunikationseinrichtung (6) Daten, insbesondere medikamentenspezifische Daten, von der Speicherservereinheit (SS) oder der Anwendungsservereinheit (AS) empfangen und Zwischenspeichern, um sie auf Abfrage oder bei Bedarf an ausgewählte medizinische Geräte (5) weiterzusenden.

9. Verfahren nach einem der Ansprüche 6 bis 8, **dadurch gekennzeichnet, dass** das erste Netzwerk vom Typ CAN und das zweite sowie dritte Netzwerk vom Typ WLAN und/oder LAN ausgewählt wird.

## Claims

1. System for controlling a data transfer to and/or from a plurality of medical devices (5), wherein the plurality of medical devices (5) is subdivided into individual groups (5a) each with at least one medical device (5), wherein each group (5a) of medical devices (5) is directly connected on a first data transfer hierarchy level (1) with a communication device (6) for the transfer, storage and control of data arranged on a second data transfer hierarchy level (2) via a first network (7), wherein the first network (7) is a direct line between the communication device (6) and the medical devices (5), and means are provided which are designed such that a plurality of these communication devices (6) exchanges data with a common central server device (8) for storage, control and data transfer arranged on a third data transfer hierarchy level (3), wherein the means are a second network (9) which is self-sufficient and independent of the first network (7) and which directly connects the communication devices (6) to the common central server device (8) arranged on the third data transfer hierarchy level (3), wherein each communication device (6) has at least one first storage unit (6a) and a first control unit (6b) in order to buffer data coming and received from the medical devices (5) of the group (5a), in particular device-specific and patient-related data of patients who are connected to the medical devices (5) and to send them in a bundled manner to a storage server unit (SS) provided in the central server device (8) and wherein the communication device (6) regularly retrieves and continuously checks an operational status of each medical device (5) which is connected to the communication device (6) and automatically performs an update of a database of the respective medical device (5) as soon as the operational status of the medical device (5) permits an update.

2. System according to claim 1, **characterised in that** the central server device (8) comprises an application server unit (AS) in addition to the storage server unit (SS), wherein the application server unit (AS) is connected to application operating units (10a, 10b, 10c) on a fourth data transfer hierarchy level (4) by means of a third network (11).

3. System according to claim 2, **characterised in that** the first storage unit (6a) and the first control unit (6b) of the communication device (6) are suitable for buffering data coming and received from the storage server unit (SS) or the application server unit (AS), in particular medication-specific data and sending them to selected medical devices (5) upon request or as required.

4. System according to any one of the preceding claims 2 or 3, **characterised in that** the third network (11) connects the application operating units (10a, 10b, 10c) to the application server unit (AS) by means of a web interface and a web browser.

5. System according to any one of the preceding claims, **characterised in that** the medical devices (5) are insulin pumps.

6. Method for controlling a data transfer to and/or a plurality of medical devices (5), wherein the plurality of medical devices (5) are subdivided into individual groups (5a) each with at least one medical device (5), wherein each group (5a) of medical devices (5) on a first data transfer hierarchy level transfers and controls data from/to a communication device (6) arranged on a second data transfer hierarchy level (2) via a first network (7), wherein the first network (7) is a direct line between the communication device (6) and the medical devices (5) and a plurality of these communication devices (6) transfers and controls data from/to a common central server device (8) arranged on a third data transfer hierarchy level (3) via a second network (9), which is self-sufficient and independent of the first network (7), wherein data received in each communication device (6) in at least a first storage unit (6a) and by means of a first control unit (6b) that are sent from the medical devices (5) of the group (5a), in particular device-specific and patient-related data of patients who are connected to the medical devices (5) are buffered and sent in a bundled manner to a storage server unit (SS) provided in the central server device (8) and wherein the communication device (6) regularly retrieves and continuously checks an operational status of each medical device (5) which is connected to the communication device (6) and automatically performs an update of a database of the respective medical device (5) as soon as the operational status of the medical device (5) permits an update.

7. Method according to claim 6, **characterised in that** the central server device (8) is equipped with an application server unit (AS) in addition to the storage server unit (SS), wherein the application server unit (AS) is connected to application operating units (10a, 10b, 10c) on a fourth data transfer hierarchy level (4) via a third network (11) and is operated, controlled as well as regulated in terms of data writing and data reading by the application operating units.

8. Method according to claim 7, **characterised in that** the first storage unit (6a) and the first control unit (6b) of the communication device (6) receive and buffer data, in particular medication-specific data from the storage server unit (SS) or the application server unit (AS) in order to send them upon request or as required to selected medical devices (5).

9. Method according to any one of claims 6 to 8, **characterised in that** the first network is selected from the CAN type and the second and third network are selected from the WLAN and/or LAN type.

## Revendications

1. Système de commande d'une transmission de données vers et/ou depuis une pluralité d'appareils médicaux (5), dans lequel la pluralité d'appareils médicaux (5) est divisée en groupes individuels (5a) avec respectivement au moins un appareil médical (5), dans lequel chaque groupe (5a) est relié directement à des appareils médicaux (5) sur un premier niveau hiérarchique de transmission de données (1) avec un dispositif de communication (6) agencé sur un deuxième niveau hiérarchique de transmission de données (2) pour la transmission, l'enregistrement et la commande de données par le biais de respectivement un premier réseau (7), dans lequel le premier réseau (7) est une ligne directe entre le dispositif de communication (6) et les appareils médicaux (5), et des moyens sont prévus qui sont réalisés pour qu'une pluralité de ces dispositifs de communication (6) échange des données avec un dispositif serveur central (8) commun agencé sur un troisième niveau hiérarchique de transmission de données (3) pour l'enregistrement, la commande et la transmission de données, dans lequel les moyens sont un deuxième réseau (9), lequel est autonome et indépendant du premier réseau (7), et lequel relie directement les dispositifs de communication (6) au dispositif serveur central (8) commun agencé sur le troisième niveau hiérarchique de transmission de données (3), dans lequel chaque dispositif de communication (6) présente au moins une première unité de stockage (6a) et une première unité de commande (6b) pour enregistrer temporairement des données provenant et reçues des appareils médicaux (5) du groupe (5a), en particulier des données de patients propres aux appareils et se rapportant aux patients, qui sont en relation avec les appareils médicaux (5), et les transmettre groupées à une unité serveur de stockage (SS) prévue dans le dispositif serveur central (8), et dans lequel le dispositif de communication (6) interroge en permanence et vérifie continuellement un état de fonctionnement de chaque appareil médical (5), qui est relié au dispositif de communication (6), et procède tout seul à une mise à jour d'une base de données de l'appareil médical (5) respectif, dès que l'état de fonctionnement de l'appareil médical (5) permet une mise à jour.

2. Système selon la revendication 1, **caractérisé en ce que** le dispositif serveur central (8) comprend, en plus de l'unité serveur de stockage (SS), une unité serveur d'applications (AS), dans lequel l'unité serveur d'applications (AS) est reliée à des unités de commande d'application (10a, 10b, 10c) sur un quatrième niveau hiérarchique de transmission de données (4) au moyen d'un troisième réseau (11).

3. Système selon la revendication 2, **caractérisé en ce que** la première unité de stockage (6a) et la première unité de commande (6b) du dispositif de communication (6) sont adaptées pour enregistrer temporairement des données provenant et reçues de l'unité serveur de stockage (SS) ou de l'unité serveur d'applications (AS), en particulier des données propres aux médicaments, et les transmettre sur demande ou en cas de besoin à des appareils médicaux (5) sélectionnés.

4. Système selon l'une quelconque des revendications précédentes 2 ou 3, **caractérisé en ce que** le troisième réseau (11) relie les unités de commande d'application (10a, 10b, 10c) à l'unité serveur d'applications (AS) au moyen d'une interface Web et d'un navigateur Web.

5. Système selon l'une quelconque des revendications précédentes, **caractérisé en ce que** les appareils médicaux (5) sont des pompes à insuline.

6. Procédé de commande d'une transmission de données vers et/ou une pluralité d'appareils médicaux (5), dans lequel la pluralité d'appareils médicaux (5) est divisée en groupes individuels (5a) avec respectivement au moins un appareil médical (5), dans lequel chaque groupe (5a) transmet et commande des données à des appareils médicaux (5) sur un premier niveau hiérarchique de transmission de données depuis/vers un dispositif de communication (6) agencé sur un deuxième niveau hiérarchique de transmission de données (2) par le biais de respectivement un premier réseau (7), dans lequel le premier réseau (7) est une ligne directe entre le dispositif de communication (6) et les appareils médicaux (5), et une pluralité de ces dispositifs de communication (6) transmet et commande des données depuis/vers un dispositif serveur central (8) commun agencé sur un troisième niveau hiérarchique de transmission de données (3) par le biais d'un deuxième réseau (9), lequel est autonome et indépendant du premier réseau (7), dans lequel des données reçues dans chaque dispositif de communication (6) dans au moins une première unité de stockage (6a) et au moyen d'une première unité de commande (6b), qui sont envoyées par les appareils médicaux (5) du groupe (5a), en particulier des données de patients propres aux appareils et se rapportant aux patients, qui sont en relation avec les appareils médicaux (5), sont enregistrées temporairement et transmises groupées à une unité serveur de stockage (SS) prévue dans le dispositif serveur central (8), et dans lequel le dispositif de communication (6) interroge en permanence et vérifie continuellement un état de fonctionnement de chaque appareil médical (5), qui est relié au dispositif de communication (6), et procède tout seul à une mise à jour d'une base de données de l'appareil médical (5) respectif, dès que l'état de fonctionnement de l'appareil médical (5) permet une mise à jour.

7. Procédé selon la revendication 6, **caractérisé en ce que** le dispositif serveur central (8) est équipé, en plus de l'unité serveur de stockage (SS), d'une unité serveur d'applications (AS), dans lequel l'unité serveur d'applications (AS) est reliée à des unités de commande d'application (10a, 10b, 10c) sur un quatrième niveau hiérarchique de transmission de données (4) par le biais d'un troisième réseau (11) et est servi, commandé et réglé en écriture de données et en lecture de données par les unités de commande d'application.

8. Procédé selon la revendication 7, **caractérisé en ce que** la première unité de stockage (6a) et la première unité de commande (6b) du dispositif de communication (6) reçoivent et enregistrent temporairement des données, en particulier des données propres aux médicaments, de l'unité serveur de stockage (SS) ou de l'unité serveur d'applications (AS) pour les transmettre sur demande ou en cas de besoin à des appareils médicaux (5) sélectionnés.

9. Procédé selon l'une quelconque des revendications 6 à 8, **caractérisé en ce que** le premier réseau de type CAN et le deuxième ainsi que le troisième réseau de type R-LAN et/ou LAN est sélectionné.
